# EUROPÄISCHE PATENTSCHRIFT

(11) **EP 1 129 778 B1**
(45) Veröffentlichungstag und Bekanntmachung des Hinweises auf die Patenterteilung: **05.04.2006**
(21) Anmeldenummer: 01104822.0
(22) Anmeldetag: 27.02.2001
(51) Int. Cl.: B01L 3/00, G01N 33/48, G01N 27/28

(54) **System zur Bestimmung von Analytkonzentrationen in Körperflüssigkeiten**
System for determining the concentration of an analyte in body fluids
Système de détermination de la concentration d'un analyte dans des fluides corporels

(30) Priorität: 03.03.2000 DE 10010587
(43) Veröffentlichungstag der Anmeldung: 05.09.2001
(73) Patentinhaber: Roche Diagnostics GmbH, 68305 Mannheim (DE); F. HOFFMANN-LA ROCHE AG, 4070 Basel (CH)
(72) Erfinder: Effenhauser, Carlo, Dr., 69469 Weinheim (DE)

(56) Entgegenhaltungen:
- EP-A- 0 138 152
- DE-A- 19 602 861

## Beschreibung

Die vorliegende Erfindung betrifft ein System zur Bestimmung der Konzentration mindestens eines Analyten in einer Flüssigkeit, insbesondere einer Körperflüssigkeit. Das System besitzt einen ersten Körper und mindestens einen zweiten Körper von denen mindestens einer eine Vertiefung auf seiner Oberfläche beinhaltet und die Körper so zusammengefügt werden, daß die Vertiefung durch eine Fläche des jeweils anderen Körpers zumindest zum Teil verschlossen wird, so daß ein Kanal gebildet wird. Der erste und / oder der zweite Körper beinhaltet einen Austauschbereich, der entweder im Bereich der Vertiefung, oder dem der Vertiefung gegenüberliegenden Teil des anderen Körpers angeordnet ist, so daß über den Austauschbereich Stoffe aus der umgebenden Flüssigkeit aufgenommen werden können. Das System weist weiterhin einen Sensor auf, mit dem die Konzentration mindestens eines Analyten in dem Kanal ermittelt werden kann. Außerdem besitzt das System mindestens ein integriertes Reservoir das in Verbindung mit dem Kanal steht.

Im Stand der Technik sind eine Vielzahl unterschiedlicher Techniken zur Detektion von Analytkoazentrationen in Körperflüssigkeiten bekannt. Liegt die Körperflüssigkeit extrakorporal vor, so kann eine Analytbestimmung auf konventionellem Wege mit einem klinischen Analyzer erfolgen. Für häufig zu vermessende Analyten, wie z. B. den Glucosegehalt von Blut, haben sich im Stand der Technik tragbare Vorrichtungen, sogenannte Blutzuckermeßgeräte durchgesetzt. Ein Nachteil der genannten Analysemethoden liegt jedoch darin, daß zunächst eine Körperflüssigkeit entnommen werden muß, was den Anwendungsbereich im Regelfall auf Einzelmessungen beschränkt. In einigen Bereichen der Medizin, insbesondere in dem Bereich des Diabetesmonitoring, ist es jedoch von großem Vorteil, eine kontinuierliche oder zumindest quasi kontinuierliche Überwachung des Glucosespiegels durchzuführen. Hierdurch können einerseits drohende hypoglykämische Zustände, die zum Tod des Patienten führen können, rechtzeitig erkannt werden als auch andererseits eine Warnung vor hyperglykämischen Zuständen erfolgen, die in der Regel mit Langzeitschäden (Erblindung, Gangräne, usw.) verbunden sind. Es sind daher in letzter Zeit erhebliche Anstrengungen unternommen worden, eine kontinuierliche Überwachung der Blutglucosekonzentration zu ermöglichen. Eine Forschungsrichtung strebt eine nichtinvasive Messung der Blutglucosekonzentration z.B. durch Messung der glucoseabhängigen Streuung und /oder Absorption von Infrarotstrahlung an. Aufgrund sehr ungünstiger Signal-Rauschverhältnisse und schwer kontrollierbarer physiologischer Einflußgrößen konnte jedoch bislang noch kein serienreifes Produkt basierend auf einem solchen Meßverfahren entwickelt werden. Eine andere Entwicklungsrichtung basiert auf der Verwendung von Sensoren, die direkt in den Körper implantiert werden, um vor Ort Messungen durchzuführen. Ein großes Problem in diesem Technologiebereich liegt jedoch in der erheblichen Drift der verwendeten Sensoren. Dieses Problem liegt u. a. daran, daß die Sensoren direkt oder über eine Membran in Kontakt mit Gewebe und Bestandteilen der Körperflüssigkeit in Kontakt kommen. Geeignete Membranen können dieses Problem zwar verringern, jedoch findet trotzdem eine Alterung der Sensormaterialien statt, die zu einer Drift führen, welche über eine Dauer von mehreren Tagen nur schwer kompensierbar ist. Exemplarisch für die vorstehend genannte Technologie wird an dieser Stelle auf das Dokument US-5,855,801 verwiesen.

Die Probleme direkt implantierter Sensoren wurden durch die Mikrodialyse, die Ultrafiltration und die Mikroperfusion weitestgehend gelöst. Bei der Mikrodialyse wird eine Perfusionsflüssigkeit durch einen Katheter geleitet und es erfolgt eine Analytbestimmung in dem aus dem Katheter austretenden Dialysat. Hieraus ergeben sich zahlreiche Erfordernisse im Bereich des Fluidhandlings und Mikrodialyseanordnungen sind im Vergleich zu implantierten Sensoren größer, weil unter anderem Perfusat und Dialysat aufbewahrt werden müssen. Andererseits stellt jedoch die Mikrodialysetechnik das heutzutage verläßlichste Verfahren dar, um Analytkonzentration in vivo zu überwachen.

Im Stand der Technik sind eine Reihe von Mikrodialysesonden bekannt, für die an dieser Stelle stellvertretend lediglich auf die in der deutschen Patentschrift DE 33 42 170 beschriebene Anordnung verwiesen wird. Die in diesem Patent beschriebene Anordnung ist im Handel unter der Bezeichnung CMA 60 Mikrodialysekatheter erhältlich. Wie aus dem Patent ersichtlich wird, sind einer Verkleinerung der Anordnung, bedingt durch die notwendigen Herstellverfahren, Grenzen gesetzt. Eine Verkleinerung ist jedoch dringend notwendig, um einer weiteren Verbreitung der Technologie Vorschub zu leisten. Dies ist vorteilhaft, weil kleinere Mikrodialysesonden wesentlich leichter und für den Patienten weniger traumatisch in den Körper eingeführt werden können.

Außerdem können kleinere Vükrodialysesonden mit weniger Flüssigkeit betrieben werden, so daß eine Verkleinerung der Flüssigkeitsreservoirs möglich ist.

In dem Artikel "A µTAS based on microdialysis for on-line monitoring of clinically relevant substances" S. Böhm, W. Othuis. P. Bergveld in: Micro Total Analysis Systems '98, D.J. Harrison, A. v.d. Berg, Eds., Kluwer Academic Publishers, Dordrecht 1998 wird eine miniaturisierte Mikrodialyseanordnung beschrieben. Die Anordnung besitzt einen mikrotechnisch hergestellten Teil, der einen Sensor und Fluidkanäle sowie einen Aufnahmebereich für die eigentliche Mikrodialysesonde beinhaltet. Die beschriebene Mikrodialysesonde besitzt einen inneren Kanal, durch den Perfusionsflüssigkeit einströmt und am Ende dieses Kanales in einen äußeren Kanal austritt. Die Mikrodialyse erfolgt durch die Membran des äußeren Kanales und das gebildete Dialysat wird dem Sensor zugeführt. Während mit dieser Anordnung bereits eine relativ starke Miniaturisierung erreicht wird, bleiben jedoch eine Reihe von Problemen ungelöst. In erster Linie baut die beschriebene Anordnung auf dem konventionellen Prinzip von Mikrodialysesonden aus konzentrischen Röhren auf, bei dem eine innere Röhre von einer äußeren Röhre umgeben ist, die in Fluidkommunikation miteinander stehen. Dies wirft nicht nur herstellungstechnische Probleme auf, sondern setzt auch einer Miniaturisierung Grenzen. Weiterhin ist die vorstehend genannte Anordnung nachteilig, da der Mikrodialysekatheter in einen Halter eingeklebt werden muß. Dies ist herstellungstechnisch nachteilig und die Übergänge der Fluidkanäle fuhren zu Problemen. Es wurde nämlich festgestellt, daß derartige Fluidübergänge zur Verschlechterung der Antwortfunktion der Signale führen. Dies resultiert daraus, daß die Übergänge Totvolumina einfiihren.

Eine weitgehende Lösung der genannten Probleme wird durch Systeme gemäß der DE 196 02 861 erreicht. Die in diesem Dokument beschriebenen Ausführungsformen vermeiden Querschnittsveränderungen in der Meßstrecke und somit daraus resultierende Beeinflussungen der Antwortfunktion des Systems. Weiterhin ermöglichen die beschriebenen Vorrichtungen eine Miniatuhsierung und besitzen trotzdem die bereits vorstehend beschriebenen Vorteile einer Mikrodialyse. In der DE 196 02 861 wird weiterhin ausgeführt, daß es sehr leicht möglich sei, Sensorelemente zu integrieren, da der Kanal vor dem Einbringen der Sensorelemente schon mit einer für den Analyten permeablen Membran bedeckt sei. Hierdurch wird ein Verschluß oder eine Kontamination des Kanals durch ein Einbringen der Sensorelemente vermieden. Die hierdurch bedingte Bauweise besitzt jedoch den Nachteil, daß der Analyt aus der Probeflüssigkeit einen noch größeren Difusionsweg zurücklegen muß, als dies bei der Mikrodialyse ohnehin schon der Fall ist. Um ein Sensorsignal hervorzurufen, muß der Analyt zunächst durch die Membran in die Trägerflüssigkeit diffundieren und darauf noch aus der Trägerflüssigkeit heraus durch eine Membran zum Sensor. Ein weiterer Nachteil der in der DE 196 02 861 beschriebenen Systeme liegt darin, daß die Trägerflüssigkeit, in der der Analyt aufgenommen wird, dem Sensorsystem über Öffnungen von Extern zugeführt werden muß. Eine derartige Konnektierung eines miniaturisierten Devices mit Schläuchen bedeutet nicht nur zusätzliche Herstellschritte, sondern wirft auch Dichtittkeitsprobleme auf. Ein unkontrolliertes Eindringen von Luftblasen in das System ist nachteilig da hierdurch sowohl der Stoffaustausch im Mikrodialysebereich als auch die Signalerzeugung im Detektionsbereich beeinflußt wird.

Die Aufgabe der vorliegenden Erfindung war es, ein miniaturisiertes Analysesystem vorzuschlagen, das die bestehenden Probleme des Standes der Technik vermeidet. Insbesondere war es Ziel der vorliegenden Erfindung, ein herstellungstechnisch günstiges und hinreichend miniaturisierbares System vorzuschlagen. Weiterhin war es Aufgabe der Erfindung ein integriertes System zu schaffen, was weitestgehend autark arbeitet und soweit als möglich auf den Anschluß von externen Reservoiren verzichtet. Hierdurch können bestimmte Fertigungsschritte vermieden werden, die Größe des Systems kann reduziert werden und aus Anschlußkonnektierungen resultierende Probleme werden reduziert bzw. vermieden.

Die vorliegende Aufgabe wird durch ein System gelöst, bei dem durch das Zusammenfügen von mindestens zwei Körpern ein Kanal entsteht, der zumindest in einem Teilbereich einen Austauschbereich besitzt, so daß Stoffe aus einer umgebenden Flüssigkeit/Körperflüssigkeit aufgenommen werden können. Darüber hinaus besitzt das System mindestens ein integriertes Reservoir, das in Verbindung mit dem Kanal steht.

Das erfindungsgemäße System dient zur Bestimmung der Konzentration mindestens eines Analyten in einer Flüssigkeit/Körperflüssigkeit. Die Bezeichnung Analyt umfaßt im Rahmen der vorliegenden Erfindung sämtliche möglichen Analyten, wie z. B. Glucose, Lactat, Proteine, Elektrolyte und Neurotransmitter. Der Begriff "Körperflüssigkeit" soll im Rahmen der vorliegenden Erfindung ebenfalls sämtliche möglichen Körperflüssigkeiten wie insbesondere interstitielle Flüssigkeit, Blut und Hirnflüssigkeit umfassen. Das System ist in erster Linie zur in-vivo Diagnostik beim Menschen konzipiert ist, es sollen jedoch auch andere Anwendungen, z. B. bei Tieren mit umfaßt sein.

Im Rahmen der vorliegenden Erfindung werden die Begriffe Dialyse, Dialysemembran etc. sowohl für Ausführungsformen verwendet, bei denen über eine Membran ein Stoffaustausch zwischen dem Außenraum und einer Perfusionsflüssigkeit stattfindet (d.h. Mikrodialysesysteme), als auch für Systeme, bei denen durch die Membran eine Filtration der das System umgebenden Körperflüssigkeit erfolgt (im allgemeinen als Ultrafiltration bezeichnet).

Einen wesentlichen Aspekt der vorliegenden Erfindung stellt der Aufbau eines Grundkörpers aus mindestens zwei Körpern dar, von denen mindestens einer eine Vertiefung besitzt und die Körper so zusammengesetzt werden, daß ein Kanal entsteht. So können sowohl Fertigung als auch Miniaturisierung vereinfacht werden. Insbesondere kann durch diesen integrierten Aufbau auf einfache Weise erreicht werden, daß zwischen dem Austauschbereich und dem Meßbereich keine Fluidübergänge mit Kavitäten, Hinterschneidungen oder dergleichen auftreten, die zu einer Signalverschlechterung führen. Jeder Fluidübergang erzeugt fertigungsbedingt einen Bereich, der nicht direkt durchströmt wird, in den jedoch durch Randströmungen Teile des Hauptstromes eindringen.

Die Körper aus denen der Grundkörper aufgebaut wird, insbesondere der/die mit Vertiefung können beispielsweise aus Silizium nach den bekannten Verfahren der Mikrobearbeitung von Silizium hergestellt werden. Unter Herstellungs- und Kostengesichtspunkten ist jedoch eine Fertigung der Körper aus Kunststoffen, Metallen oder Keramiken bevorzugt. Insbesondere kann der bzw. die Körper auf einfache und kostengünstige Weise aus Polymeren im Spritzgußverfahren hergestellt werden, wobei z. B. Vertiefungen im Grundkörper für Reservoirs und Kanäle direkt im Spritzgußverfahren eingebracht werden können. Es ist jedoch auch möglich, einen Kunststoffkörper durch Prägetechniken und dergleichen nachträglich zu bearbeiten. Kunststoffe, die zu diesem Zweck eingesetzt werden können, sind zum Beispiel Polymethylmethacrylat und Polycarbonat. Es sollen auch solche Ausführungsformen umfaßt sein, bei denen auf den Grundkörper nachträglich eine Beschichtung aufgebracht wird. Dies kann günstig oder notwendig sein, um die Oberfläche zu passivieren, ihr eine geeignete Oberflächenspannung zu verleihen oder aber um Elektroden aufzubringen. Verfahren zum Aufbringen von derartigen Beschichtungen sind beispielsweise Aufdampfen oder Aufsputtern von Metallen wie Gold, Silber und Aluminium. Da diese Verfahren im Stand der Technik hinlänglich bekannt sind, wird an dieser Stelle nicht näher auf sie eingegangen.

Der Grundkörper besitzt einen Austauschbereich durch den Stoffe aus der umgebenden Körperflüssigkeit in den Kanal aufgenommen werden können. Diese Aufnahme kann einerseits durch Vorbeiführen einer Flüssigkeit (Perfusionsflüssigkeit) an einer Membran (d.h. Mikrodialyse) oder durch Aufnahme von Flüssigkeit in den Kanal durch eine Membran (Ultrafiltration) durch Anlegen eines Druckgradienten erfolgen. Weiterhin ist es möglich, im Austauschbereich Perforationen vorzusehen, durch die Stoffe aus dem Außenraum aufgenommen werden können. Eine solche Vorgehensweise wird im allgemeinen als Mikroperfusion bezeichnet.

Eine Besonderheit der vorliegenden Erfindung besteht darin, daß der Austauschbereich nicht wie im Stand der Technik üblich, durch ein System aus koaxialen Röhren gebildet wird, wie zum Beispiel in der DE 33 42 170 beschrieben, sondern daß der Austauschbereich von einem nach oben offenen Kanal im Grundkörper gebildet wird, der von einer Membran oder einem perforierten Bereich abgedeckt ist. Wird Perfusionsflüssigkeit durch den Kanal hindurchgeführt, während sich der Austauschbereich in Kontakt mit einer Körperflüssigkeit befindet, so nimmt die Perfusionsflüssigkeit Substanzen aus der Körperflüssigkeit auf.

Im Falle der Mikrodialyse und Ultrafiltration richtet sich die Art der Substanzen, die aufgenommen werden, nach der Beschaffenheit der Membran, insbesondere nach deren Porenweite. Prinzipiell können für die Membran die im Stand der Technik verwendeten Materialien, wie zum Beispiel Polycarbonat, Celluloseacetat, Polysulfon, verwendet werden. Eine Beschreibung geeigneter Membranen findet sich beispielsweise in der DE 196 02 861 und US 4,832,034.

Die Membran dient vor allem dazu, molekulare Stoffe, die die Analyse stören, bzw. eine Alterung des Sensors hervorrufen, auszuschließen. Dementsprechend sollten Stoffe mit einem Molekuiargewicht > 10.000 Dalton durch die Membran ausgeschlossen werden.

Im Falle der Mikroperfusion besitzt der Austauschbereich Perforationen, durch die direkt Fluid zwischen dem Außenraum und dem Kanal ausgetauscht werden können. Die einzelnen Perforationen können dabei einen Öffnungsquerschnitt von wenigen Mikrometern bis hin zu etwa 0,5 mm aufweisen. Zur Vorgehensweise im Rahmen der Mikroperfusion wird an dieser Stelle auf das Dokument US 5,097,834, hingewiesen.

Während herkömmliche Mikrodialysekatheter eine Austauschfläche besitzen, die im wesentlichen eine zylindrische Gestalt aufweist, besitzt der Austauschbereich der vorliegenden Erfindung eine im wesentlichen planare Fläche oberhalb des Kanales als Austauschfläche. Diese nach absoluten Maßstäben verringerte Austauschfläche wird dadurch kompensiert, daß der Kanal im Austauschbereich sehr klein, insbesondere flach, ausgeführt werden kann, was zu einem günstigen Oberflächen/Volumen-Verhältnis führt und eine schnelle prozentuale Anreicherung mit Analyt gewährleistet. Konventionelle Mikrodialysesonden weisen eine aktive Katheterlänge im Bereich weniger Zentimeter auf. Mit einem Austauschbereich gemäß der vorliegenden Erfindung ist eine Länge von einem Zentimeter und darunter möglich. Durch entsprechende Formengebung des Kanales im Austauschbereich, zum Beispiel Schlingen oder Meander, kann die notwendige Länge des Körpers weiter verringert bzw. die aktive Länge vergrößert werden. Wie bereits ausgeführt, wird bei der vorliegenden Erfindung ein Austauschbereich durch Abdecken eines Kanales im Dialysebereich mit einer Membran oder einer perforierten Struktur erzielt. Hierzu kann die Membran oder die perforierte Struktur beispielsweise auf den Dialysebereich aufgeklebt oder aufgesiegelt werden.

Der Austauschbereich des Grundkörpers weist vorzugsweise eine längliche Gestalt auf, so daß er die Form eines Stabes besitzt. Der Stab ist vorzugsweise monolithisch mit dem.Grundkörper (bzw. mit einem Körper des Grundkörpers) verbunden. Der mit dem Grundkörper verbundene Teil des Stabes wird als proximales Ende bezeichnet, wohingegen das gegenüberliegende Ende als distales Ende bezeichnet wird. Das distale Ende kann beispielsweise spitz ausgeführt sein, so daß eine Einführung in den Körper erleichtert wird. Andererseits ist eine Spitze nicht notwendig, wenn ein sogenanntes Applikationsbesteck verwendet wird, um den Dialysebereich in den Köper einzuführen. Im Stand der Technik existieren eine Vielzahl verschiedener Typen von Applikationsbestecken, auf die an dieser Stelle nicht näher eingegangen wird. Es sei lediglich stellvertretend auf die Dokumente WO 97/14468 (TFX Medical Inc.) und WO 95/20991 (CMA Microdialysis Holding AB) hingewiesen.

Der Kanal im Austauschbereich ist so ausgeführt, daß er einen Einlaß und einen Auslaß aufweist, die beide vorzugsweise am proximalen Ende des Dialysebereiches angeordnet sind. In diesem Zusammenhang ist erwähnenswert, daß eine strikte Unterscheidung zwischen dertrKanal des Austauschbereiches (Austauschkanal) und den übrigen Kanälen des Grundkörpers kaum möglich ist, da diese Kanäle ineinander übergehen. Eine Definition des Austauschkanales ist allenfalls dahingehend möglich, daß man diese Bezeichnung für den Kanalteil verwendet, der von der Dialysemembran bzw. der perforierten Struktur überdeckt ist und somit zum Austauschvorgang beiträgt. Flüssigkeit, die aus dem Austauschkanal austritt, kann direkt oder über einen Kanal einem auf dem Grundkörper befindlichen Meßbereich zugeführt werden. Zur Reduktion des Zeitversatzes zwischen der Meßwertaufnahme, also dem Austauschvorgang und der Messung, wird der Kanal in aller Regel möglichst kurz gewählt werden. Der Kanal wird seinerseits durch einen nach oben offenen Kanal im Grundkörper gebildet, der durch eine Deckplatte abgedeckt ist. Diese Deckplatte kann prinzipiell aus dem gleichen Material wie der Grundkörper bestehen, also insbesondere aus Kunststoffen. Von dem Begriff "Deckplatte" sollen auch solche Ausführungsformen umfaßt sein, die im allgemeinen Sprachgebrauch aufgrund ihrer geringen Dicke eher als Folie bezeichnet werden. Die Deckplatte sollte aus einem flüssigkeitsundurchlässigen Material bestehen, ansonsten brauchen im Normalfall jedoch keine speziellen Forderungen an sie gestellt zu werden. Zu erwähnen ist noch die Auswahl eines Materials, das mit dem Dialysat bzw. Mikroperfusat kompatibel ist und zu keinen Veränderungen führt, die die Konzentration des zu bestimmenden Analyten oder die Analyse als solche beeinflussen. Für bestimmte Analyten kann es jedoch vorteilhaft sein, wenn die Deckplatte oder zumindest ein Teil davon im Bereich des Kanales luftdurchlässig ist. Dies ist insbesondere für den Nachweis von Glucose mittels Glucoseoxidase günstig, da die Glucose zur Detektion in der Regel mit Luftsauerstoff oxidiert wird. In einem solchen Fall ist es günstig, bereits die Perfusionsflüssigkeit mit Luftsauerstoff zu sättigen. Hierfür kann die Deckplatte ganz oder teilweise aus einem sauerstoff durchlässigen Material wie z.B. einem Silikon hergestellt werden.

Im Meßbereich des Grundkörpers ist mindestens ein Sensor zur Detektion eines Analyten angeordnet. Zur Detektion von Glucose kann z. B. eine Metallelektrode verwendet werden, die an ihrer Oberfläche mit Glucoseoxidase oder einem glucoseoxidasehaltigen Reagenzgemisch beschichtet ist. Vorzugsweise befindet sich diese Elektrode auf der Deckplatte, so daß sie beim Zusammenfügen von Deckplatte und Grundkörper im Meßbereich oberhalb des Kanales stromabwärts des Austauschbereiches angeordnet ist. Die Aufbringung einer solchen Elektrode auf die Deckplatte kann beispielsweise durch Aufdampfen oder Aufsputtern eines Metallstreifens erfolgen, der nachfolgend mit Glucoseoxidase bzw. einem Reagenzgemisch beschichtet wird. Zur Aufbringung von Reagenzgemischen auf Elektroden eignen sich z.B. Siebdruckverfahren. Grundsätzlich kann die Elektrode jedoch auch als separates Bauteil auf dem Grundkörper angebracht werden.

Zusätzlich zu der beschriebenen Meßelektrode weist das System noch eine Gegenelektrode auf, die in entsprechender Weise auf die Deckplatte aufgebracht sein kann. Bei einer weiter unten näher ausgeführten Anordnung kann auf die Beschichtung einer Elektrode mit einem Reagenzgemisch verzichtet werden, da dem Dialysat eine flüssige Lösung von Glucoseoxidase beigemischt wird. Dieses Meßverfahren und eine in diesem Rahmen geeignete Sensoranordnung ist in der EP B 0 393 054 beschrieben. Neben den vorstehend genannten elektrischen Meßzellen können im Rahmen der vorliegenden Erfindung auch optische Meßzellen verwendet werden. Hierzu kann beispielsweise im Meßbereich ein mit dem Analyten farbbildendes Reagenzsystem angeordnet sein, wie es für Teststreifen zur Blutglucosemessung bekannt ist. Ein optischer Sensor, der zur Messung der Glucosekonzentration geeignet ist, wird beispielsweise in der EP A 0693 271 beschrieben. Eine weitere Möglichkeit, das System mit einem Sensor auszustatten, besteht in Meßzellen gemäß der US 5.393.401. Diese Meßzellen können in den Grundkörper integriert werden, indem sie als separate Bauteile eingesetzt werden oder indem in dem Körper eine sich in Richtung des Kanales verjüngende Ausnehmung eingebracht wird, in der sich das Sensorsystem befindet.

Ein wesentlicher Aspekt eines erfindungsgemäßen Systems ist es, daß zwar einerseits Fluide transportiert werden, um eine Detektion zu ermöglichen, wie dies bei Mikrodialyse, Ultrafiltration und Mikroperfusion der Fall ist, daß jedoch andererseits hierzu notwendige Flüssigkeiten oder Reservoirs zur Aufnahme von Flüssigkeiten so weit als möglich, vorzugsweise vollständig in den Grundkörper integriert sind, so daß auf Flüssigkeitsanschlüsse verzichtet werden kann. Dieses wird erreicht, indem Reservoirs in den Grundkörper integriert werden. Für eine Durchführung einer Mikrodialyse ist es beispielsweise vorteilhaft, ein Reservoir für Perfusionsflüssigkeit und / oder ein Reservoir zur Aufnahme von Dialysat nach der Analyse vorzusehen. Für eine Ultrafiltration, bei der im Normalfall keine Perfusionsflüssigkeit eingesetzt wird, kann erfindungsgemäß ein Abfallreservoir zur Aufnahme von Ultrafiltrat stromabwärts der Meßstelle vorgesehen werden. Sowohl für ein Mikrodialyse, als auch für eine Ultrafiltration kann es notwendig oder vorteilhaft sein, Hilfsfluide wie Enzymlösungen (insbesondere Glucoseoxidaselösung) oder Kalibrationsflüssigkeit zu verwenden. Auch Reservoirs zur Aufnahme dieser Hilfsflüssigkeiten können vorteilhaft in den Grundkörper integriert werden. Jeder Einführungskanal in den Grundkörper, der vermieden werden kann, ist dabei von Vorteil, da entsprechende Arbeitsschritte zur Konnektierung, Herstellung und Sterilisierung, sowie Probleme mit Fluidübergängen, wie Dichtigkeit und Signalverschlechterungen entfallen.

Bei einem besonders bevorzugten System sind alle notwendigen Reservoirs in dem Grundkörper integriert, so daß auf externe Fluidanschlüsse vollständig verzichtet werden kann. Ein solches System weist einen abgeschlossenen Flüssigkeitspfad auf.

Ein System gemäß der vorliegenden Erfindung weist, sofern mit ihm eine Mikrodialyse oder eine Mikroperfusion durchgeführt wird, ein Reservoir für Perfusionsflüssigkeit auf, das direkt oder über einen Perfusatkanal mit dem Austauschbereich verbunden ist. Vorzugsweise ist das Reservoir für Perfusionsflüssigkeit und ggf. der Perfusatkanal in den Grundkörper integriert. Es sind jedoch auch Ausführungsformen möglich, bei dem das Reservoir separat vom Grundkörper, beispielsweise in Form eines Kunststoffbeutels vorliegt, der direkt oder über einen Kanal an den Austauschbereich angeschlossen wird. Aufgrund der bereits erwähnten herstellungstechnischen Nachteile diskret aufgebauter Systeme ist es jedoch vorteilhaft, ein Reservoir für Perfusionsflüssigkeit im Grundkörper vorzusehen. Dies kann durch eine Vertiefung in einem Körper erfolgen, der durch Abdecken mit einem weiteren Körper verschlossen wird. In diesem Zusammenhang ist zu erwähnen, daß es von der vorliegenden Erfindung auch umfaßt sein soll, wenn statt eines einzelnen Körpers zur Abdeckung des ersten Körpers zwei oder mehr diskrete Körper, beispielsweise Platten, verwendet werden. Dies kann vorteilhaft sein, wenn das Verschließen des Reservoirs und das Abdecken des Meßbereiches in separaten Schritten erfolgen soll.

Zum Transport von Perfusionsflüssigkeit durch den Austauschbereich und hin zum Sensorbereich ist erfindungsgemäß eine Pumpe vorgesehen. Eine solche Pumpe kann beispielsweise im Druckbetrieb arbeiten und somit Flüssigkeit aus dem Reservoir für Perfusionsflüssigkeit herausdrücken oder aber sie kann auch im Saugbetrieb arbeiten und Flüssigkeit durch das System hindurchziehen. Weiterhin kann eine Pumpe beispielsweise so angeordnet sein, daß sie Flüssigkeit aus dem Fluidreservoir herauszieht und dem Austauschbereich zuführt. Letzere Variante kann analog einer konventionellen Schlauchpumpe ausgeführt sein, bei der durch ein von außen angreifendes Rollenelement Flüssigkeit durch Zusammenquetschen eines zusammendrückbaren Bereiches des Fluidkanales verschoben wird. Ein solcher zusammenquetschbarer Teil läßt sich beispielsweise im Bereich des Perfusatkanales realisieren, wenn einer der Körper in diesem Bereich deformierbar gestaltet ist.

Eine Druckpumpe kann im Rahmen der vorliegenden Erfindung dadurch realisiert werden, daß das System im Bereich des Perfusatreservoirs zusammendrückbar gestaltet ist und von außen ein mechanischer Druck auf diesen Bereich ausgeübt wird. Weiterhin ist es möglich, das Innere des Perfusatreservoirs mit einem Gasdruck zu beaufschlagen, um Perfusionsflüssigkeit herauszudrücken. Entsprechende Systeme, sind beispielsweise im Bereich der "implanted delivery devices" gebräuchlich. Exemplarisch sei an dieser Stelle jedoch auf das Dokument WO99/41606 (Fig. 7) aus dem Bereich der Mikrodialyse, verwiesen.

Wie bereits erwähnt, können im Rahmen der vorliegenden Erfindung auch Saugpumpen eingesetzt werden, mit denen ein Unterdruck stromabwärts des Meßbereiches angelegt wird, um Flüssigkeit durch den Austauschbereich hindurch in den Sensorbereich zu ziehen. Entsprechende Sauganordnungen sind insbesondere aus dem Bereich des on-line monitoring von Körperflüssigkeiten mittels der Ultrafiltration bekannt. Insbesondere wird in diesem Zusammenhang auf die in dem Artikel "Ultrafiltrate sampling device for continous monitoring"; D. Muskone, K. Venema, J. Korff; in Medical a. biological engineering and computing, 1996, 34, Seiten 290-294, und in der US 4,777. 953 beschriebene Anordnungen verwiesen.

Bei diesen Anordnungen wird zunächst ein Unterdruckreservoir geschaffen, z.B. durch das Auziehen einer Spritze und die Auffüllung des Unterdruckreservoir wird durch eine Flußrestriktion verzögert, so daß über einen Zeitraum von mehreren Tagen ein im wesentlichen konstanter Fluß erzielt wird.

Im Rahmen der vorliegenden Erfindung werden vorzugsweise Kanäle mit einem Durchmesser im Bereich von 10-1000µm eingesetzt. Bei Kanallängen im Bereich einiger Zentimeter ergibt sich, daß zur Erzielung linearer Flußraten von etwa 1 cm / min Drücke im Bereich weniger Millibar hinreichend sind. Dies bedeutet jedoch auch, daß bereits geringe Druckschwankungen (z.B. Lageänderungen des Systems) den Flüssigkeitstransport ungewollt beeinflussen können. Dementsprechend ist es im Rahmen der vorliegenden Erfindung vorteilhaft, Flußbegrenzungen einzubauen, die dazu führen, daß ein Flüssigkeitstransport erst beim Vorhandensein einer größeren Druckdifferenz erfolgt. Solche Flußbegrenzungen können vorteilhaft in den Grundkörper integriert werden, indem Kanalbereiche vorgesehen werden, bei denen der Kanal auf einer Länge von mehreren Zentimetern auf einen geringeren Querschnitt (beispielsweise unterhalb 100 µm²) begrenzt ist. Solche Verjüngungen können mit den weiter oben genannten Produktionstechniken für den Grundkörper auf einfache Weise erzielt werden.

Ein erfindungsgemäßes System weist weiterhin eine mit dem Sensor verbundene Auswerteeinheit auf, die zur Umwandlung von Sensorsignalen in Konzentrationswerte des Analyten dienen. Derartige Auswerteeinheiten sind im Stand der Technik, beispielsweise für elektrochemische Blutzuckermeßsgeräte, hinlänglich bekannt, so daß an dieser Stelle nicht näher darauf eingegangen zu werden braucht. Es sei jedoch angemerkt, daß die Auswerteeinheit nicht direkt mit dem Sensor verbunden zu sein braucht sondern daß beispielsweise ein Sender vorgesehen werden kann, der mit dem Sensor verbunden ist und Signale an einen Empfänger sendet, der seinerseits mit einer Ausweneeinheit verbunden ist. Eine solche räumliche Trennung zwischen dem Grundkörper und der Auswerteeinheit kann in mehrfacher Hinsicht vorteilhaft sein. Unter anderem wird hierdurch erreicht, daß das System von Ballast (Auswerteeinheit, Display) befreit wird. Dies erhöht insbesondere den Tragekomfort des Systems für einen Patienten.

Die vorliegende Erfindung wird anhand einiger Figuren näher erläutert.
- Figur 1:: Aufsicht, vertikaler Schnitt durch den Grundkörper und Explosionszeichnung einer ersten Ausführungsform
- Figur 2:: Aufsicht auf und vertikaler Schnitt durch einen Grundkörper einer zweiten Ausführungsform eines Mikrodialysesystems
- Figur 3:: Darstellung unterschiedücher Schichten-Kombinationen zur Generierung des Grundkörpers.
- Figur 4:: Druckpumpenanordnung

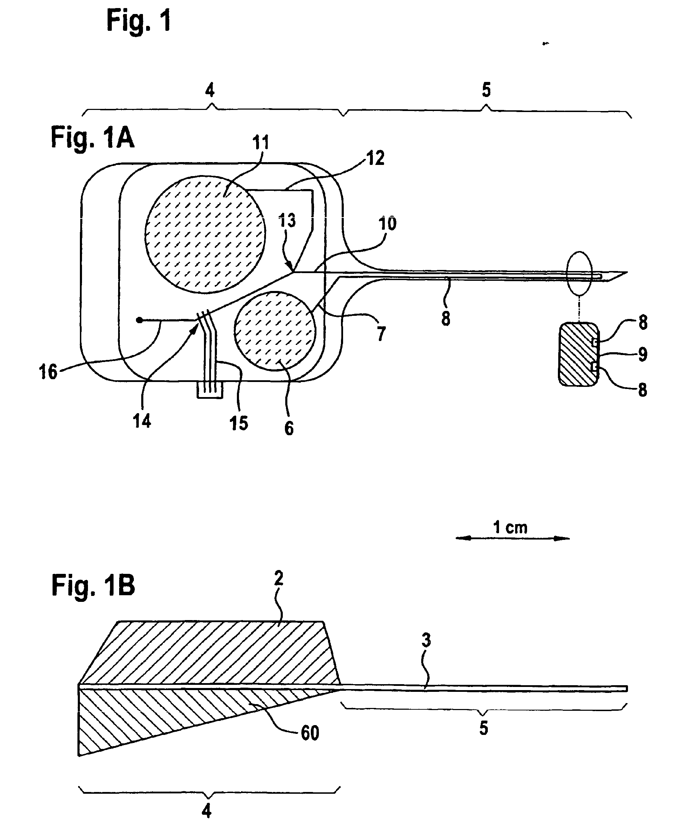

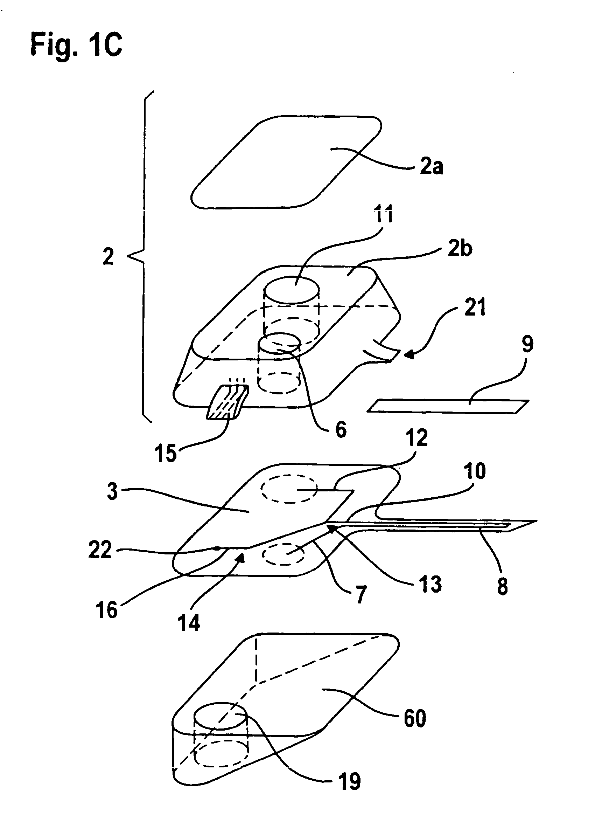

In der Figur 1 ist eine erste Ausführungsform eines Grundkörpers für ein Mikrodialysesystem gezeigt. In der Figur 1A ist zu erkennen, daß der Grundkörper zwei Bereiche aufweist. Der erste Bereich (4) beinhaltet den Meßbereich und der zweite Teil (5) den Dialysebereich. Bei der dargestellten Ausfuhrunesform befindet sich im ersten Bereich (4) eine Vertiefung in einem ersten Körper (Oberteil, 2), die beim Zusammenfügen mit einem zweiten Körper (3) ein Reservoir (6) für Perfusionsflüssigkeit bildet. Im vorliegenden Fall weist das Reservoir ein Volumen von 250 µl auf und enthält, wie im Bereich der Mikrodialyse gebräuchlich, Ringerlösung. Perfusionsflüssigkeit kann aus diesem Reservoir durch Anlegen eines Druckes durch den Perfusatkanal (7) in den Dialysekanal (8) gedrückt werden. Im dargestellten Beispiel besteht der Dialysekanal (8) aus einer nach oben offenen Vertiefung im Dialysebereich der zweiten Körpers. Dieser Kanal erstreckt sich vom proximalen Ende des Dialysekanales bis in die Nähe des distalen Endes, macht dort eine Biegung und verläuft zurück in den proximalen Teil des Dialysebereiches. Wie aus der Ausschnittszeichnung, die einen Querschnitt durch den vorderen Teil des Dialysebereiches zeigt, hervorgeht, ist der Kanal (8) durch eine Membran (9) abgedeckt, die eine Dialyse ermöglicht, wenn sich der Dialysebereich in Kontakt mit einer Körperflüssigkeit befindet. Vorzugsweise kann die Membran auch über diesen Bereich hinausgehen und sich über die gesamte Oberseite des zweiten Körpers erstrecken. Hierdurch lassen sich Dichtigkeitsprobleme an der Nahtstelle erster Körper- zweiter-Körper-Membran vermeiden. Um Zugänge zu den Sensoren und Reservoirs bereitzustellen können Ausnehmungen/Durchbrechungen in der Membran vorgesehen werden.
An den Auslaß des Dialysekanales schließt sich im ersten Bereich (4) der Dialysatkanal (10) an, durch den das im Dialysebereich gebildete Dialysat in den Meßbereich (14) transportiert wird. Im Meßbereich befindet sich ein Sensor (15), mit dem eine Analytkonzentration im Dialysat bestimmt werden kann. Ein hierfür geeigneter Sensor ist beispielsweise in der EP B 0 603 154 (AVL Medical Instruments AG) beschrieben. In dem in Figur 1 dargestellten Fall wird hingegen ein einfacher Metallelektrodensensor verwendet, wie er in der EP B 0 393 054 beschrieben ist. Dieser Sensor arbeitet ohne ein immobilisiertes Enzym, so daß sonst häufig auftretende Probleme, wie eine Signaldrift, wegfallen. Bei Anwendung dieses Sensors wird im vorliegenden Fall dem Dialysat eine Lösung von Glucoseoxidase beigemischt, so daß in der Flüssigkeit eine Oxidation der Glucose stattfindet, wobei Wasserstoffperoxid entsteht. Das Wasserstoffperoxid ist das eigentliche Agens, das vom Sensor detektiert wird. Die Lösung von Glucoseoxidase kann beispielsweise als Perfusionsflüssigkeit verwendet werden, so daß die Mikrodialyseanordnung mit nur einer Flüssigkeitskomponente auskommt. Um einen Austritt von glucoseoxidasehaltiger Flüssigkeit in den Körperinnenraum jedoch auf jeden Fall ausschließen zu können, ist es bevorzugt, die Glucoseoxidaselösung wie in Figur 1 dargestellt, dem bereits gebildeten Dialysat beizumischen. In Figur 1 ist hierfür eine Mischstelle (13) vorgesehen. Vorteilhaft kann der Bereich des Dialysatkanales, der stromabwärts der Mischstelle (13) liegt, durch ein sauerstoffdurchlässiges Material abgedeckt sein, so daß die sauerstoffverbrauchende Oxidation der Glucose vollständig ablaufen kann. Nachdem das Dialysat an dem Meßbereich (14) vorbeigeströmt ist, gelangt es in einen Abfallkanal (16) und wird von dort aus in ein Abfallreservoir (19) eingeleitet, das sich im Unterteil (60) befindet. Aus der Figur 1 ist durch die Angabe eines Zentimetermaßstabes zu erkennen, wie stark das Mikrodialysesystem durch Verwendung eines integrierten Grundkörpers miniaturisiert werden kann.

Figur B zeigt einen Querschnitt durch den Grundkörper. In dem Oberteil (2) befinden sich die in Figur A dargestellten Reservoirs (6, 11). Der zweite Körper (3) besitzt Vertiefungen, die durch Verschließen mit dem Oberteil ein Kanalsystem bilden. Der zweite Körper besitzt weiterhin ein Durchgangsloch, das in fluidischer Verbindung mit dem Abfallreservoir des Unterteils (60, dritter Körper) steht, so daß sich nach Zusammenfügen von zweitem und drittem Körper ein Reservoir ausbildet. Der äußeren geometrischen Form des Dialysebereichs und des Unterteils sind kaum Grenzen gesetzt, so daß man sie den anatomischen Bedürfnissen anpassen kann.

Figur 1C zeigt eine Explosionsdarstellung des Mikrodialysesystems. Aus dieser Darstellungsweise ist zu erkennen, daß das Oberteil (2) seinerseits vorteilhaft aus zwei Teilen (2a, 2b) zusammengesetzt sein kann. Der untere Teil (2b) besitzt Bohrungen, die im Zusammenwirken mit dem oberen Teil (2a) und dem zweiten Körper (3) Reservoirs für Perfusionsflüssigkeit (6) und Glucoseoxidaselösung (11) bilden. Der Teil (2b) trägt an seiner Unterseite ferner Elektroden (15), die die Meßzelle bilden. Zur Herstellung einer fluiddichten Verbindung besitzt der Teil (2b) weiterhin eine Nase (21), welche im zusammengesetzten Zustand oberhalb der Membran (9) zu liegen kommt. Aus Figur 1C ist weiterhin die Mikrostrukturierung des zweiten Körpers (3) mit den auf seiner Oberfläche angeordneten Rillen zu erkennen, welche im Zusammenwirken mit dem Teil (2b) die Kanäle (7, 8, 10, 12, 16) bereitstellen. Der Körper (3) hat weiterhin eine durchgehende Bohrung (22), durch die Flüssigkeit aus dem Kanal (16) ins Abfallreservoir (19) gelangt. Zum Fluidtransport in dem Mikrodialysesystem kann das Abfallreservoir mit einem Unterdruck beaufschlagt werden, so daß Flüssigkeit aus den Reservoirs (6, 11) durch die Kanäle gezogen wird. Die Flußrate kann durch den Strömungswiderstand des Kanalsystems insbesondere durch die verwendeten Kanalquerschnitte eingestellt werden.

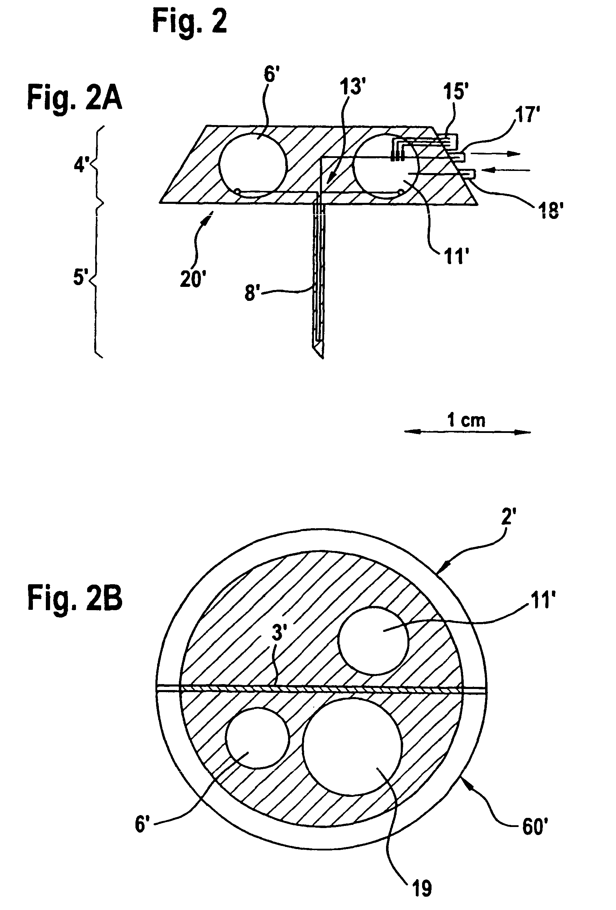

In Figur 2 ist ein Grundkörper einer zweiten Ausfiihrungsform dargestellt, dessen Aufbau analog zu der in Figur 1 dargestellten Ausfiihrungsform ist. Für die einzelnen Einheiten wurden Bezugszeichen verwendet, die zu denen in Figur 1 korrespondieren und lediglich durch einen hochgesetzten Strich unterschieden wurden. Ein wesentlicher Unterschied der beiden Ausfühnmgsformen besteht darin, daß der Aufbau bei der zweiten Ausfiihnmgsform so gewählt wurde, daß ein senkrechtes Einführen des Dialysebereiches (5') in den Körper möglich ist und der erste Teil des Grundkörpers (4') mit der Fläche (20') auf dem Körper aufliegt. Der Flüssigkeitstransport findet in der in Figur 2 dargestellten Anordnung analog zur Figur 1 statt, indem Perfusionsflüssigkeit aus dem Reservoir (6') durch den Dialysekanal (8') gelangt, ihr Glucoseoxidaselösung aus dem Reservoir (11') an der Mischstelle (13') zugemischt wird und schließlich zum Sensor (15') gelangt. Der Flüssigkeitstransport erfolgt bei dieser Ausführungsform, indem an den Anschluß (17') ein Unterdruck angelegt wird, so daß Flüssigkeit aus dem Reservoir (6') durch den Dialysekanal und zum Sensor transportiert wird. Durch Regelung des Einströmens von Flüssigkeit oder Luft in das Reservoir (11') durch den Einlaß (18') kann die Menge an zugemischter Glucoseoxidaselösung geregelt werden.

In Figur 2B ist eine Aufsicht gezeigt. Es ist zu erkennen, daß der Bereich 4' aus zwei Hälften zusammengesetzt ist, zwischen denen sich eine Platte (3') befindet. Die Platte (3', zweiter Körper) besitzt Vertiefungen, die mit der einen Hälfte (erster Körper; 2') und / oder der anderen Hälfte (dritter Körper. 60') zusammen Fluidkanäle bilden. Durch eine beiderseite Strukturierung der Platte (3'), können eine Vielzahl von Kanalgeometrien (3') realisiert werden. Durch eine beiderseitige Strukturierung im Austauschbereich kann auch eine vergrößerte Austauschfläche erzielt werden. In Figur 2B ist weiterhin ein Abfallbehältnis (19) dargestellt, das stromabwärts des Sensors angeordnet ist.

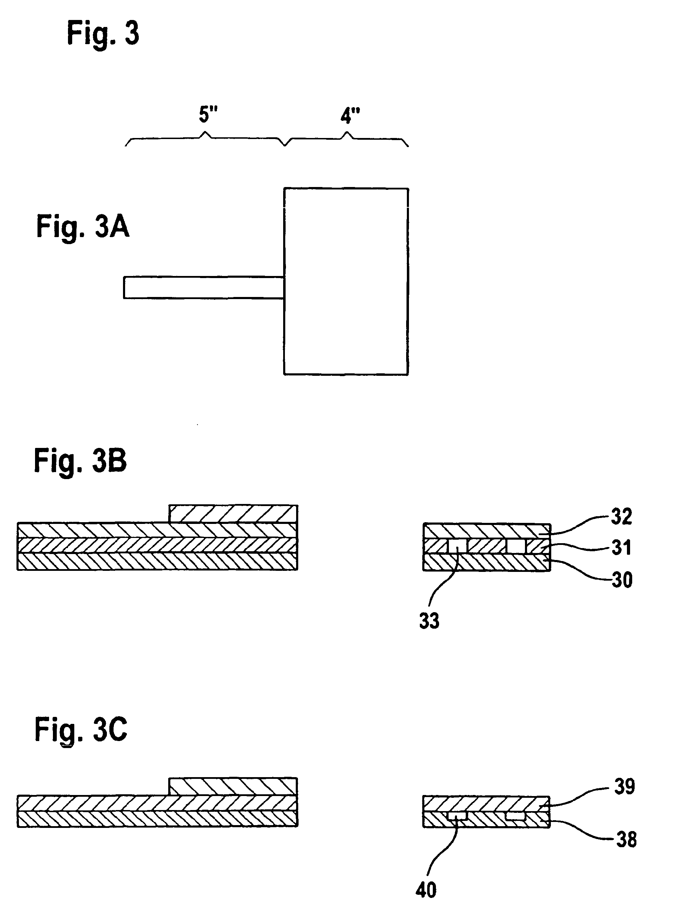

Figur 3 zeigt ein erfindungsgemäßes System, das aus mehreren Schichten aufgebaut ist. Wie aus Figur 3A hervorgeht, weist auch dieses System einen Austauschbereich (5") zur Einführung in den Körper, sowie einen Bereich (4") auf, in dem der Meßbereich angeordnet ist. In den Figuren 3B-3C sind Querschnitte längs des Austauschbereiches (linke Spalte) als auch quer durch den Austauschbereich (rechte Spalte) dargestellt. Diese Figuren zeigen unterschiedliche Schichtenaufbauten zur Realisierung der Kanäle. In Figur 3B ist zunächst eine unstrukturierte Platte (30) mit planer Oberfläche verwendet worden, auf die eine Platte (31) mit Ausnehmungen aufgebracht ist. Durch Zusammenwirken dieser beiden Platten ergibt sich ein erster Körper, der auf seiner Oberfläche nach oben offene Kanäle aufweist. Dieser Kanal bzw. die Kanäle werden im dargestellten Fall durch eine Membran (32) abgedeckt, so daß sich ein geschlossener Kanal (33) ergibt.

In Figur 3C schüeßlich ist eine Ausführungsform gezeigt, bei der ein oberflächenstrukturierter Körper (38) mit nach oben offenen Vertiefungen verwendet wurde, der durch eine ebene Membran (39) abgedeckt ist. Auch hier entstehen durch das Zusammenwirken der Körper eine oder mehrere Fluidkanäle (40).

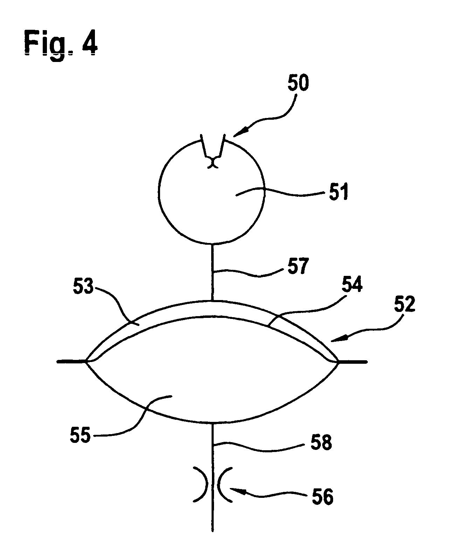

Figur 4 zeigt in schematischer Darstellung eine Druckpumpe, wie sie bevorzugt in Systemen gemäß der vorliegenden Erfindung eingesetzt werden kann. Die Druckpumpe besitzt zunächst ein Einlaßventil (50), durch das mit einer Vorrichtung, z.B. mit einem Kolbenprober bzw. einer Spritze ein Gasdruck in den Druckbehälter (51) gegeben werden kann. Nach dem Herausziehen der Pipette bzw. eines Druckanschlusses aus dem Ventil schließt dieses, so daß der Gasdruck in dem Druckbehälter erhalten bleibt. Der Druckbehälter ist über einen Kanal (57) mit einem Membransystem (52) verbunden, das eine flexible impermeable Membran (54) aufweist. Im für eine Anwendung bereiten Ausgangszustand befindet sich die Membran (54) in einer Lage, in der der Hohlraum (53) klein oder sogar verschwindend ist und der mit Perfusionsflüssigkeit gefüllte Bereich (55) groß ist. Mit aus dem Druckbehälter durch den Kanal (57) einströmendem Gas wird die Membran verschoben und Perfusionsflüssigkeit aus dem Reservoir (55) in den Kanal (58) hineingedrückt. Stromabwärts des Membransystems befindet sich ein Flußbegrenzer (56), beispielsweise in Form einer Verjüngung des Kanales, der die pro Zeiteinheit aus dem Reservoir (55) austretende Flüssigkeitsmenge begrenzt und konstant hält. Auf diese Weise ist es möglich, einen über mehrere Tage konstanten Fluß von Perfusionsflüssigkeit zur Verfügung zu stellen, um damit einen Austauschbereich zu beaufschlagen.

Die in Figur 4 dargestellte Pumpenanordnung ist für ein sandwichartig aufgebautes System gemäß der vorliegenden Erfindung produktionstechnisch besonders günstig, denn die obere Halbschale der Membrananordnung kann als Vertiefung in dem ersten Körper und die untere Halbschale durch eine Vertiefung des zweiten Körpers gebildet werden und die Membran (54) kann auf einfache Weise zwischen diesen beiden Halbschalen bzw. Körpern durch Einklemmen oder Einkleben befestigt werden.

## Patentansprüche

1. System zur Bestimmung der Konzentration mindestens eines Analyten in einer Flüssigkeit, insbesondere einer Körperflüssigkeit beinhaltend
einen ersten Körper (2,2'), sowie mindestens einen weiteren, zweiten Körper (3,3'), von denen mindestens einer eine Vertiefung auf seiner Oberfläche besitzt und erster ünd zweiter Körper so zusammengefügt sind, daß die Vertiefung zumindest teilweise durch eine Fläche des anderen Körpers verschlossen wird, so daß ein Kanal (7, 8, 10, 8') gebildet wird, wobei
- der erste und / oder der zweite Körper einen Austauschbereich beinhaltet über den Stoffe aus der umgebenden Flüssigkeit in den Kanal aufgenommen werden können und
- das System weiterhin einen stromabwärts vom Austauschbereich im Kanal angeordneten Sensor (15, 15') aufweist, mit dem die Konzentration eines Analyten ermittelt werden kann,
wobei das System mindestens ein integriertes Reservoir (6, 11, 6', 11', 19) besitzt, das in Verbindung mit dem Kanal steht.

2. System gemäß Anspruch 1, das einen länglichen, in den die Flüssigkeit enthaltenden Körper einführbaren Austauschbereich (5, 5') aufweist, in dem ein Teil des Kanales verläuft.

3. System gemäß Anspruch 1, bei dem das mindestens eine Reservoir durch eine Vertiefung in dem ersten oder /und zweiten Körper gebildet wird.

4. System gemäß Anspruch 1, das eine Pumpe zur Bewegung von Flüssigkeit mittels Druck oder Unterdruck durch den Kanal beinhaltet

5. System gemäß Anspruch 4, bei dem die Pumpe in die Anordnung aus erstem und zweitem Körper integriert ist.

6. System gemäß Anspruch 1 bei dem das mindestens eine Reservoir oder ein zusätzliches Reservoir zur Aufnahme von Flüssigkeit dient und stromabwärts vom Sensor angeordnet ist.

7. System gemäß Anspruch 1, das einen Begrenzer zur Begrenzung eines Flusses in dem Kanal aufweist.

8. System gemäß Anspruch 1, bei dem der Körper mit Vertiefung sowohl eine Platte (30) als auch eine auf der Platte befestigte Folie (31) mit Ausnehmungen beinhaltet, so daß die Vertiefung durch Zusammenwirken von Platte und Folie gebildet wird.

9. System gemäß Anspruch 1, bei dem alle für die Funktion des Systems notwendigen Reservoirs in den ersten und / oder zweiten Körper integriert sind.

10. System gemäß Anspruch 4, bei dem die Pumpe in das System integriert ist:

## Claims

1. System for determining the concentration of at least one analyte in a liquid, in particular in a body fluid comprising
a first member (2, 2') and at least one additional second member (3, 3') of which at least one has a recess on its surface and the first and second member are joined together in such a way that the recess is at least partially closed by a surface of the other member to form a channel (7, 8, 10, 8') wherein
the first and/or the second member contain an exchange region by means of which substances can be taken up from the surrounding fluid into the channel and
the system additionally has a sensor (15, 15') located downstream of the exchange region in the channel which can be used to determine the concentration of an analyte,
wherein the system has at least one integrated reservoir (6, 11, 6', 11', 19) which is connected to the channel.

2. System as claimed in claim 1, which has an elongate exchange region (5, 5') in which a part of the channel is located and which can be inserted into the body, holding the liquid.

3. System as claimed in claim 1, in which the at least one reservoir is formed by a recess in the first or/and the second member.

4. System as claimed in claim 1, which contains a pump for moving liquid through the channel by means of pressure or underpressure.

5. System as claimed in claim 4, in which the pump is integrated into the arrangement comprising the first and second member.

6. System as claimed in claim 1, in which the at least one reservoir or an additional reservoir is used to hold liquid and is located downstream of the sensor.

7. System as claimed in claim 1, which has a restrictor to limit flow in the channel.

8. System as claimed in claim 1, in which the member having the recess contains a plate (30) and a foil (31) with recesses that is attached to the plate such that the recess is formed by interaction of the plate and foil.

9. System as claimed in claim 1, in which all reservoirs necessary for the function of the system are integrated into the first and/or second member.

10. System as claimed in claim 4, in which the pump is integrated into the system.

## Revendications

1. Système pour la détermination de la concentration d'au moins un analyte dans un liquide, en particulier dans un fluide corporel, renfermant
un premier corps (2, 2'), et au moins un deuxième corps supplémentaire (3, 3'), au moins un possédant un renfoncement sur sa surface, et le premier et le deuxième corps étant joints l'un à l'autre de telle sorte que le renfoncement est fermé au moins en partie par une surface de l'autre corps, si bien qu'un canal (7, 8, 10, 8') est formé,
- le premier et/ou le deuxième corps renfermant une zone d'échange par laquelle des substances provenant du liquide environnant peuvent venir se loger dans le canal, et
- le système présentant en outre un capteur (15, 15') disposé dans le canal en aval de la zone d'échange, avec lequel la concentration d'un analyte peut être déterminée,
- le système possédant au moins un réservoir intégré (6, 11, 6', 11', 19) qui entre en liaison avec le canal.

2. Système selon la revendication 1, qui présente une zone d'échange oblongue (5, 5') qui s'introduit dans le corps contenant le liquide, dans laquelle s'étend une partie du canal.

3. Système selon la revendication 1, dans lequel ledit au moins un réservoir est formé par un renfoncement dans le premier et/ou dans le deuxième corps.

4. Système selon la revendication 1, qui renferme une pompe pour le déplacement du liquide à travers le canal à l'aide d'une pression ou d'un vide.

5. Système selon la revendication 4, dans lequel la pompe est intégrée dans l'agencement constitué par le premier et le deuxième corps.

6. Système selon la revendication 1, dans lequel ledit au moins un réservoir ou un réservoir supplémentaire sert à la réception du liquide et est disposé en aval du capteur.

7. Système selon la revendication 1, qui présente un limiteur pour limiter un écoulement dans le canal.

8. Système selon la revendication 1, dans lequel le corps comprenant un renfoncement renferme aussi bien une plaque (30) qu'une feuille (31) fixée sur la plaque, comprenant des évidements, de telle sorte que le renfoncement est formé par la coopération de la plaque et de la feuille.

9. Système selon la revendication 1, dans lequel tous les réservoirs requis pour le fonctionnement du système sont intégrés dans le premier et/ou dans le deuxième corps.

10. Système selon la revendication 4, dans lequel la pompe est intégrée dans le système.
